(19) **Europäisches Patentamt / European Patent Office / Office européen des brevets**

(11) **EP 2 888 572 B1**

(12) # FASCICULE DE BREVET EUROPEEN

(45) Date de publication et mention de la délivrance du brevet:
**09.10.2019 Bulletin 2019/41**

(21) Numéro de dépôt: **13762077.9**

(22) Date de dépôt: **19.08.2013**

(51) Int Cl.:
*G01N 21/27* (2006.01)  *G01N 21/64* (2006.01)
*G01N 15/14* (2006.01)  *G01N 35/00* (2006.01)
*G01N 33/49* (2006.01)  *G01N 15/10* (2006.01)

(86) Numéro de dépôt international:
**PCT/EP2013/067251**

(87) Numéro de publication internationale:
**WO 2014/029743 (27.02.2014 Gazette 2014/09)**

(54) **PROCEDE DE COMPENSATION DU VIEILLISSEMENT D'UN REACTIF LORS DE MESURES DE FLUORESCENCE SUR DES PARTICULES, ET DISPOSITIF D'ANALYSE BIOLOGIQUE METTANT EN OEUVRE LE PROCEDE**

VERFAHREN ZUR KOMPENSATION DER ALTERUNG EINES REAGENS BEI FLUORESZENZMESSUNGEN AN TEILCHEN UND BIOLOGISCHER ANALYSATOR ZUR DURCHFÜHRUNG DES VERFAHRENS

METHOD FOR COMPENSATING FOR THE AGING OF A REAGENT DURING FLUORESCENCE MEASUREMENTS CARRIED OUT ON PARTICLES, AND BIOLOGICAL ANALYSIS DEVICE IMPLEMENTING THE METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **21.08.2012 FR 1257905**

(43) Date de publication de la demande:
**01.07.2015 Bulletin 2015/27**

(73) Titulaire: **Horiba ABX SAS / Horiba Ltd**
**34184 Montpellier cedex 4 (FR)**

(72) Inventeurs:
• **URANKAR, Alexandra**
  **F-34980 Saint Clement De Riviere (FR)**
• **NERIN, Philippe**
  **F-34820 Assas (FR)**
• **HARICHE, Moustapha**
  **F-34980 Combaillaux (FR)**
• **CAUET, Gilles**
  **F-34270 Fontanès (FR)**

(74) Mandataire: **IPAZ**
**18 rue de la République**
**34000 Montpellier (FR)**

(56) Documents cités:
**EP-A2- 0 678 742**    **US-A1- 2011 222 062**

• **RUUD HULSPAS ET AL: "Flow cytometry and the stability of phycoerythrin-tandem dye conjugates", CYTOMETRY PART A, vol. 75A, no. 11, 23 septembre 2009 (2009-09-23), pages 966-972, XP055062054, ISSN: 1552-4922, DOI: 10.1002/cyto.a.20799**
• **PAULI J ET AL: "An in vitro characterization study of new near infrared dyes for molecular imaging", EUROPEAN JOURNAL OF MEDICINAL CHEMISTRY, EDITIONS SCIENTIFIQUE ELSEVIER, PARIS, FR, vol. 44, no. 9, 29 janvier 2009 (2009-01-29), pages 3496-3503, XP026322174, ISSN: 0223-5234, DOI: 10.1016/J.EJMECH.2009.01.019 [extrait le 2009-01-29]**
• **RESCH-GENGER U ET AL: "How to Improve Quality Assurance in Fluorometry: Fluorescence-Inherent Sources of Error and Suited Fluorescence Standards", JOURNAL OF FLUORESCENCE, KLUWER ACADEMIC PUBLISHERS-PLENUM PUBLISHERS, NE, vol. 15, no. 3, 1 mai 2005 (2005-05-01), pages 337-362, XP019281694, ISSN: 1573-4994**

**Description**

**Domaine technique**

La présente invention concerne un procédé pour compenser la dégradation au cours du temps d'un réactif fluorescent lors de mesures de fluorescence effectuées sur des particules, dont des cellules, marquées par ce réactif.
Le domaine de l'invention est plus particulièrement mais de manière non limitative celui des dispositifs d'analyse biologique, et en particulier celui des dispositifs d'hématologie.

**Etat de la technique antérieure**

L'analyse cellulaire, et notamment l'analyse de cellules sanguines, nécessite l'utilisation de réactifs spécifiques permettant un traitement chimique primaire de l'échantillon. Diverses fonctions physico-chimiques sont réalisées telles que la dilution, la coloration ou la destruction sélective de certaines populations cellulaires (lyse différentielle).
Ces réactifs sont conditionnés dans des containers adaptés à leur utilisation dans des équipements d'analyse tels que des automates d'hématologie ou des cytomètres en flux. Il est d'usage de donner à chaque réactif une seule fonction chimique, mais la tendance actuelle est de donner plusieurs fonctions à un même réactif pour des considérations de coût ou de simplicité d'utilisation. La simplicité de manutention tant chez le fabricant du produit que chez l'utilisateur est aussi un but recherché.
Il existe néanmoins des difficultés quand on cherche à fabriquer un réactif unique offrant plusieurs fonctions physico-chimiques simultanées : les divers composés chimiques du réactif peuvent réagir entre eux, pour donner naissance à des sous-produits dont les fonctions physico-chimiques s'écartent notablement des produits d'origine. Cette modification ou dégradation peut altérer le traitement primaire de l'échantillon, notamment sanguin, et peut se traduire par un résultat d'analyse erroné.
La modification ou dégradation du réactif peut aussi dépendre de facteurs environnementaux tels que par exemple, la température ou les rayonnements électromagnétiques. En particulier, certains composés fluorescents utilisés dans des réactifs se dégradent en phase aqueuse, avec une vitesse de dégradation qui dépend de la température. C'est notamment le cas du Thiazole Orange (TO), qui est un composé fluorescent utilisé comme colorant de composantes cellulaires et destiné à s'associer à l'ADN ou l'ARN intracellulaire.
Des réactifs comprenant des composés fluorescents comme le TO sont notamment décrits dans le document FR 2 821 428. Ils peuvent être utilisés dans des systèmes d'hématologie, tel que décrits par exemple dans le document WO 2006/024716. La dégradation des composés fluorescents du réactif entraîne une diminution des niveaux de fluorescence qui peut induire des erreurs de comptage et de classification des cellules analysées.
La décomposition (ou photodécomposition) des polyméthines hétérocycliques dont fait partie le TO en présence d'oxygène moléculaire est connue. La présence d'un puissant agent oxydant comme le peroxyde d'hydrogène conduit également à la dégradation du TO avec formation d'un des produits de dégradation identifié, la benzothiazolone. Mais la dégradation du TO s'effectue même en l'absence de lumière, et l'addition d'antioxydants ou le remplacement de l'oxygène dissous par un gaz inerte comme l'argon ne ralentit pas cette dégradation. Il apparaît que la dégradation du TO dans les réactifs d'hématologie résulte d'une hydrolyse spontanée de la molécule.
Certains réactifs sont formulée de telle sorte à limiter ou au moins à ralentir cette dégradation, tels que par exemple ceux décrits dans le document US 7 638 290. Toutefois, cela n'est pas suffisant pour garantir une bonne stabilité des composés dans le temps.
Pour éviter ou freiner le phénomène de modification ou dégradation en milieu aqueux, les composés fluorescents peuvent être conservés dans un solvant organique sous forme hautement concentrée et dilués lors des mesures, tel que décrit par exemple dans le document EP 2 175 340. En effet, dans un solvant organique comme un alcool ou l'éthylène glycol, la modification ou dégradation du composé fluorescent est quasi-inexistante, ce qui est un réel avantage par rapport à un solvant aqueux.
Toutefois, cette façon de procéder présente d'autres inconvénients. Il est nécessaire de mettre en oeuvre un plus grand nombre de conteneurs au sein même de l'analyseur, ce qui entraîne également une charge de gestion plus importante du nombre de produits au sein du laboratoire d'analyse ou du laboratoire d'hématologie.
En outre, lorsqu'un réactif chimique doit être reconstitué avant son utilisation, au sein même de l'analyseur ou dans un dispositif placé à coté, la complexité de mise en oeuvre est aussi accrue puisque, in fine, il est nécessaire de procéder au mélange de deux ou plusieurs liquides dont les viscosités ne sont pas toujours égales. Cela peut poser des problèmes d'homogénéité du produit, qui peuvent conduire à un mauvais traitement de l'échantillon. Un mélangeur dont l'efficacité est plus ou moins parfaite doit être utilisé, ce qui entraîne un surcoût et un risque accru de pannes.
Un objet de la présente invention est de proposer un procédé qui permette de mesurer ou au moins d'évaluer le vieillissement d'un réactif.
L'article de R. Hulspas et al. "Flow cytometry and the stability of phycoerythrin-tandem dye conjugates", CYTOMETRY PART A, vol. 75A, no. 11, 23 septembre 2009, divulgue l'estimation du vieillissement d'un réactif fluorescent utilisé dans la cytométrie en flux en mesurant une perte de fluorescence ("PE leakage").

Un autre objet de la présente invention est de proposer un procédé qui permette de compenser des erreurs de mesure de fluorescence dues au vieillissement d'un réactif.

Un autre objet de la présente invention est de prolonger la durée de vie des réactifs utilisés.

Un autre objet de la présente invention est de proposer un marqueur représentatif de la température à laquelle a été exposé le réactif pendant sa vie, y compris durant les transports le cas échéant.

**Exposé de l'invention**

L'invention est définie par les revendications indépendantes.

Les objectifs sont atteints avec un procédé pour compenser la dégradation d'un réactif stocké en phase aqueuse comportant au moins un composé fluorescent et permettant l'identification de particules, comprenant une étape de mesure de niveau de fluorescence FLUOm(t) sur des particules marquées par ledit réactif, caractérisé en ce qu'il comprend en outre des étapes :

- d'exécution de mesures d'absorbance à au moins une longueur d'onde sur une solution dudit réactif, à un instant t proche de celui desdites mesures de niveau de fluorescence FLUOm(t), de telle sorte à déterminer au moins une densité optique courante DO(t) du réactif, et,
- de calcul d'une correction des mesures de niveau de fluorescence en utilisant ladite ou lesdites densité(s) optique(s) courante(s) DO(t) et au moins une densité optique initiale DO(0) du réactif exempt de dégradation.

Suivant des modes de mise en oeuvre :

- le réactif exempt de dégradation peut être tel qu'en sortie de production ;
- des mesures d'absorbance peuvent être exécutées à au moins une longueur d'onde correspondant à un pic d'absorption d'un composé fluorescent ;
- les particules peuvent être telles que des cellules ou comprendre des cellules.

Le réactif peut comprendre notamment :

- au moins un composé fluorescent de type polyméthine,
- du Thiazole Orange.

Suivant des modes de mise en oeuvre, le procédé selon l'invention peut comprendre des étapes de :

- calcul d'un rapport K(t) correspondant au ratio entre une densité optique initiale DO(0) et une densité optique courante DO(t), et
- calcul de niveaux de fluorescence corrigés FLUOc(t) en multipliant des niveaux de fluorescence FLUOm(t) mesurés vers l'instant de mesure t sur des particules marquées par le réactif, par le rapport K(t).

Le procédé selon l'invention peut comprendre en outre des étapes de :

- mesure de densités optiques initiales DOi(0) et de densités optiques courantes DOi(t) à plusieurs longueurs d'onde λi (correspondant par exemple aux pics d'absorption de composés fluorescents),
- calcul de rapports Ki(t) auxdites longueurs d'onde λi, et
- calcul de niveaux de fluorescence corrigés FLUOci(t) auxdites longueurs d'onde λi.

Suivant d'autres modes de mise en oeuvre, le procédé selon l'invention peut comprendre en outre des étapes de :

- détermination au préalable d'un modèle de dégradation établissant une relation entre des variations de niveau de fluorescence ΔFLUO sur des particules de référence et des variations de la densité optique du réactif ΔDO du fait de sa dégradation, et
- calcul d'un nombre de canaux de fluorescence perdus CP(t), à partir de mesures de densité optique courante DO(t), de mesures de densité optique initiale DO(0), et dudit modèle de dégradation.

Le procédé selon l'invention peut comprendre en outre des étapes de :

- détermination au préalable d'un modèle de dégradation sous la forme d'un coefficient α correspondant à un ratio entre des variations de niveau de fluorescence ΔFLUO et des variations de densité optique du réactif ΔDO,
- calcul d'un nombre de canaux de fluorescence perdus CP(t), sous la forme d'une différence entre la densité optique courante DO(t) et la densité optique initiale DO(0), multipliée par le coefficient α,
- détermination d'une valeur de référence de fluorescence <FLUO(t)> statistiquement représentative des niveaux de fluorescence FLUOm(t) mesurés,
- calcul d'un gain correctif GC(t) correspondant à une valeur de référence de fluorescence <FLUO(t)> divisée par une différence entre ladite valeur de référence de fluorescence <FLUO(t)> et le nombre de canaux de fluorescence perdus CP(t), et
- calcul de niveaux de fluorescence corrigés FLUOc(t) en multipliant des niveaux de fluorescence FLUOm(t) mesurés vers un instant de mesure t sur des particules marquées par le réactif, par le gain correctif GC(t).

Le procédé selon l'invention peut comprendre en outre une détermination d'une pluralité de modèles de dégradation à partir de mesures de densité optique à plusieurs longueurs d'onde.

Suivant un autre aspect, il est proposé un dispositif d'analyse cellulaire par cytométrie en flux, comprenant des moyens de mesure de niveau de fluorescence sur des particules marquées par un réactif, caractérisé en ce qu'il comprend en outre :

- des moyens pour effectuer des mesures d'absorbance à au moins une longueur d'onde sur une solution dudit réactif, à un instant t proche de celui desdites mesures de niveau de fluorescence, de telle sorte à déterminer au moins une densité optique courante DO(t) du réactif, et
- des moyens de calcul aptes à calculer une correction des mesures de niveau de fluorescence en utilisant ladite ou lesdites densité(s) optique(s) courante(s) DO(t) et au moins une densité optique initiale DO(0) du réactif exempt de dégradation.

Suivant des modes de réalisation, le dispositif selon l'invention peut comprendre une cuve de mesure spectrophotométrique.

La cuve de mesure spectrophotométrique peut comprendre une source de lumière polychromatique et un photorécepteur doté d'un système de filtrage optique permettant la mesure des densités optiques aux diverses longueurs d'onde centrales λi.

Suivant des modes de réalisation, le dispositif selon l'invention peut comprendre en outre un analyseur d'hématologie.

Ainsi, le procédé selon l'invention apporte un certain nombre d'avantages, tels que :

- une prolongation de la durée de vie des réactifs utilisés,
- une réduction du nombre et de la quantité de produits à approvisionner dans le laboratoire,
- la possibilité de réaliser des systèmes d'analyse plus compacts, avec un système fluidique simplifié, sans mélangeur ni conditionneur de réactif au sein de l'analyseur,
- des risques de panne réduits,
- des coûts de revient et d'exploitation plus attractifs.

**Description des figures et modes de réalisation**

D'autres avantages et particularités de l'invention apparaîtront à la lecture de la description détaillée de mises en oeuvre et de modes de réalisation nullement limitatifs, et des dessins annexés suivants :

- la figure 1 illustre la dégradation de la molécule de Thiazole Orange (TO) en produits d'hydrolyse,
- la figure 2 illustre le spectre d'absorption du TO (figure 2a), et les spectres d'absorption des produits d'hydrolyse (figures 2b et 2c),
- la figure 3 illustre l'évolution des mesures de fluorescence obtenues avec du TO sur des granulocytes, respectivement 7 semaines (figure 3a) et 16 mois (figure 3b) après la production du réactif,
- la figure 4 illustre l'absorbance d'un réactif comprenant du TO en fonction du temps, en semaines,
- la figure 5 illustre la position (en intensité) du pic de fluorescence pour des neutrophiles en fonction de l'âge du réactif de la figure 3, en jours,
- la figure 6 présente des mesures de variation de niveau de fluorescence en fonction de variations de densité optique,
- les figures 7a à 7f illustre des résultats de mesure obtenus avec le procédé selon l'invention,
- la figure 8 illustre une séquence d'opérations réalisées lors de la fabrication d'un réactif,
- la figure 9 illustre une séquence d'opérations réalisées dans un dispositif d'analyse biologique ou sanguine.

On va décrire un mode de réalisation du procédé selon l'invention appliqué à un réactif comprenant un composé fluorescent, le Thiazole Orange (TO), stocké en solution aqueuse, dont l'utilisation est par exemple décrite dans le document WO 2006/024716.

Ce mode de réalisation n'est nullement limitatif, et l'invention peut bien entendu s'appliquer à d'autres réactifs comportant par exemple plusieurs agents colorants.

Comme expliqué précédemment, l'invention permet de prolonger la durée de vie des réactifs. Dans le mode de mise en oeuvre décrit, l'utilisation du modèle de correction de l'intensité de la fluorescence permet de prolonger de plusieurs mois la durée d'utilisation du réactif, qui peut passer ainsi de six à neuf mois, ou même à douze mois sans dégradation de la performance analytique. La date de péremption des réactifs utilisés dans les analyseurs est donc prolongée d'autant.

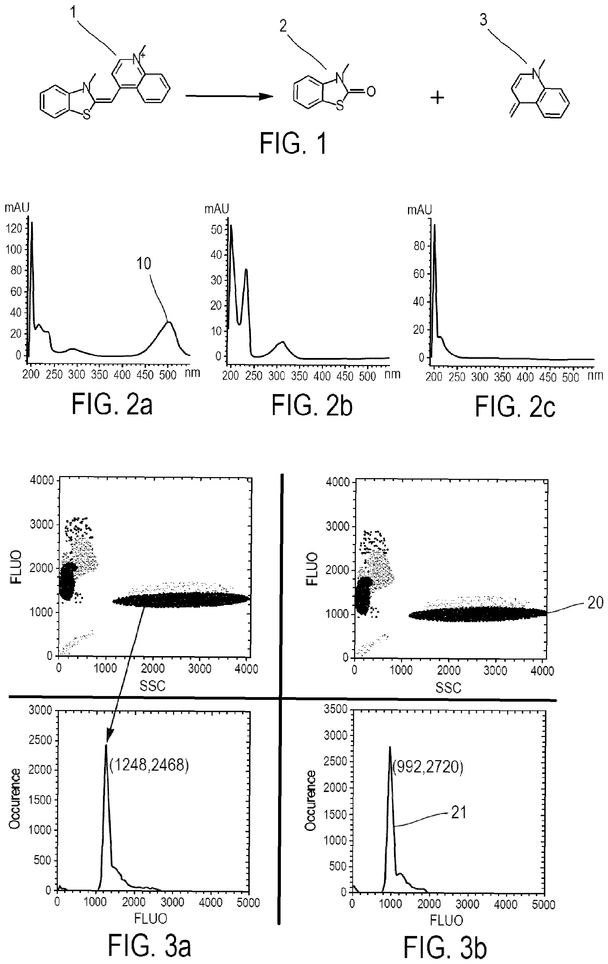

Comme illustré à la figure 1, en milieu aqueux le Thiazole Orange (TO) est instable et se dégrade progressivement. Cette dégradation résulte de la scission irréversible de la molécule de TO 1 au niveau du pont méthylénique en deux produits d'hydrolyse, soit d'une part la partie benzothiazolone (3-méthyl-2(3H)-benzothiazolone) 2, et d'autre part un dérivé de la quinoléine (1-méthyl-4-méthylène quinoléine) 3.

En référence à la figure 2, le TO présente un maximum d'absorption 10 dans le visible à une longueur d'onde de l'ordre de 500 nm (figure 2a). Or, comme illustré aux figure 2b et 2c, aucun des deux produits d'hydrolyse 1, 2 formés ne présente d'absorbance à ces longueurs d'onde.

Ainsi, suivant un aspect avantageux de l'invention, la dégradation du TO peut être précisément quantifiée en mesurant l'absorbance du réactif à des longueurs d'onde de l'ordre de 500 nm.

On rappelle que l'absorbance est définie comme une mesure de la capacité d'un milieu à absorber la lumière qui le traverse. On l'appelle également densité optique ou extinction.

La dégradation du TO est d'autant plus rapide que la température est élevée, et elle s'accompagne d'une perte de fluorescence du réactif. Ainsi à 22°C, la dégradation du Thiazole Orange quantifiée par son absorbance à 500 nm atteint environ 10 % au bout de 6 mois et peut atteindre 40 % dans le même temps à 35°C.

En référence à la figure 3, lorsque le réactif décrit dans le document FR 2 821 428 est utilisé dans un cytomètre en flux tel que celui décrit dans le document WO 2006/024716, la dégradation de la molécule entraîne une modification de ses propriétés optiques, et notamment une perte de fluorescence :

- la figure 3a présente des mesures obtenues à l'aide d'un analyseur d'hématologie avec un réactif utilisé 7 semaines après sa date de production, référencé S7 ;
- la figure 3b présente des mesures obtenues sur le même échantillon avec un réactif utilisé 16 mois après sa date de production, référencé M16.

Dans chaque cas, le graphique inférieur (Occurrence) présente un histogramme 21 des mesures de fluorescence obtenues sur des cellules marquées avec un réactif tel que décrit dans FR 2 821 428. La mesure de fluorescence (FLUO) est une mesure de fluorescence orthogonale, effectuée selon une direction perpendiculaire aux directions d'illumination et de flot de cellules, qui correspond à la mesure référencée FL1 dans le document WO 2006/024716. Dans le graphique supérieur, chaque point représente une cellule. Les points sont positionnés selon des axes correspondant, respectivement, aux mesures de fluorescence orthogonale (FLUO) et à des mesures de diffusion orthogonale (SSC) effectuées simultanément également selon une direction perpendiculaire aux directions d'illumination et de flot de cellules.

La position des cellules dans le graphe FLUO-SSC permet d'identifier les leucocytes granuleux (polynucléaires neutrophiles et éosinophiles), les lymphocytes, les érythroblastes, les monocytes, les leucocytes immatures granuleux, et les cellules à fort contenu en acides nucléiques. Cette classification est détaillée dans le document WO 2006/024716. En particulier, le nuage de points 20 correspond aux granuleux.

Avec le réactif M16, le niveau de fluorescence des leucocytes granuleux est plus faible d'environ 256 = 1258-992 canaux (ou niveaux d'intensité de fluorescence FLUO).

Dans cet exemple, l'affaiblissement de la fluorescence est gênant pour trois raisons :

- Le niveau de fluorescence du Thiazole Orange, qui est une mesure quantitative des acides nucléiques (ARN+ADN), permet la différenciation entre cellules matures et cellules immatures, comme par exemple décrit dans le document de brevet FR 2 873 813. Les cellules immatures ne sont présentes dans le sang qu'en cas de pathologie, leur identification est donc primordiale en termes de diagnostic. Un niveau de fluorescence non-stabilisé pour une lignée cellulaire pourrait conduire à l'augmentation de faux négatifs ;
- dans l'exemple de la figure 3, le biologiste voit apparaître le niveau de fluorescence des granuleux sur le canal 992 et non sur le canal 1248. Cette fluorescence au thiazole doit être sensiblement invariable pour une cellule mature donnée telle que le neutrophile (le matériel génétique ADN+ARN étant constant pour une cellule normale mature) ;
- Il peut engendrer des erreurs de classification cellulaire : ici la population des érythroblastes a été surestimée puisque une partie des lymphocytes sont identifiés et comptés dans la zone des érythroblastes.

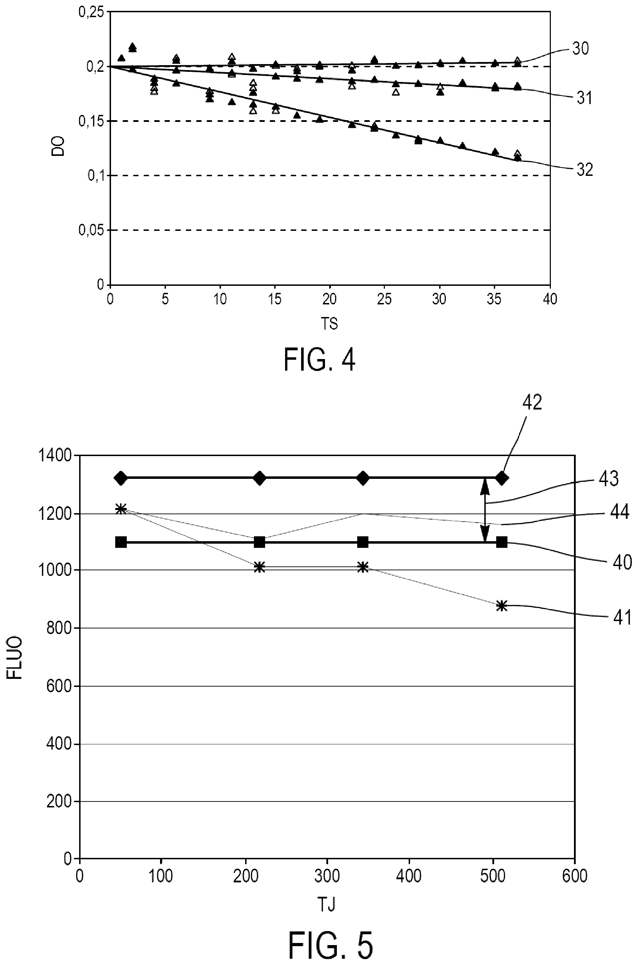

Comme mentionné précédemment, la dégradation du TO a un effet sur l'absorbance du réactif vers 500 nm. La figure 4 présente des mesures d'absorbance (DO) effectuées en fonction du temps (TS), ici en semaines, pour différentes températures :

- la courbe 30 correspond à une température de 4°C,
- la courbe 31 correspond à une température de 22°C,
- la courbe 32 correspond à une température de 35°C.

La vitesse de dégradation est donc bien fonction de la température de stockage du réactif. Par ailleurs, le fait que les bouteilles soient ouvertes ou fermées n'influence pas la cinétique, ce qui confirme que cette dégradation ne résulte pas d'un phénomène d'oxydation.

D'après la figure 4, à partir d'une mesure de densité optique (DO), il est donc possible de détecter le niveau de dégradation du réactif engendré par l'effet combiné du temps et de la température.

Il est très important de souligner qu'il s'agit d'un marqueur de la température et des conditions thermiques de stockage : dans des conditions de stockage convenables et pour un temps donné, le produit ne se dégradera pas au-delà d'une certaine limite. Il est donc possible de déterminer a posteriori si les conditions de stockage ont été respectées depuis la date de fabrication jusqu'à la date d'utilisation du produit. Cette détérioration suit l'intégrale de la température et le mécanisme de détérioration est irréversible. La mesure d'absorption indique la fraction du Thiazole Orange qui a perdu sa structure moléculaire nominale.

Avantageusement, nous avons découvert qu'il était possible de construire sur cette base des modèles de correction de la fluorescence.

La courbe 41 de la figure 5 montre l'évolution de la position (ou de l'intensité) du pic modal de fluorescence (FLUO) des leucocytes granuleux en fonction de l'âge du réactif. Les croix correspondent respectivement à des dates d'utilisation à 7 semaines, 6 mois, 12 mois et 16 mois à partir de la date de fabrication du réactif. L'axe horizontal (TJ) est en jours.

Comme on peut l'observer, les niveaux de fluorescence diminuent en fonction du temps de manière monotone, mais sans qu'il semble évident de prime abord de déterminer avec précision la loi de décroissance.

Des modèles de correction de la fluorescence ont donc été élaborés dans le cadre de l'invention.

Premier modèle de correction

Suivant premier modèle de correction, le procédé de compensation de la dégradation du réactif comprend les étapes suivantes.

Dans un premier temps, on effectue une mesure de la densité optique initiale du réactif pris isolément à sa date de fabrication, donc sans dégradation. Cette quantité est notée DO(0). Cette mesure peut être effectuée par exemple lorsque le réactif est réceptionné, ou lors de sa fabrication.

Lors de l'utilisation de l'analyseur, on effectue des mesures de la densité optique courante DO(t) du réactif pris isolément à un instant t donné. Ces mesures sont effectuées de préférence à un instant t proche du moment où sont effectuées les mesures de fluorescences, notées FLUOm(t), c'est-à-dire dans la même journée ou la même semaine. Elles peuvent être effectuées par exemple lors d'un cycle de maintenance ou de mise en route journalier ou hebdomadaire de l'analyseur sanguin.

Pour effectuer la correction, on calcule un rapport :

$$K(t) = DO(0) / DO(t).$$

Ce rapport K(t) permet de comparer DO(t) relativement à la valeur nominale de la densité optique du réactif sans dégradation DO(0).

Le rapport K(t) donne un coefficient (supérieur à un) qui est utilisé comme coefficient de correction du signal de fluorescence mesuré à la date t d'utilisation du réactif, FLUOm(t), ce qui permet de calculer le niveau de fluorescence corrigé

$$FLUOc(t) = K(t) \times FLUOm(t).$$

L'effet de ce premier modèle de correction est illustré à la figure 5 : Les points de la courbe 44 correspondent aux valeurs mesurées du pic de fluorescence des granuleux de la courbe 41 multipliés par le coefficient de correction K.

On peut observer que cette opération permet de replacer l'intensité de fluorescence entre les deux limites de tolérance haute 42 et basse 43 qui permettent à l'algorithme une identification précise de cette population de granuleux.

Ce premier modèle de correction est basé sur une hypothèse de proportionnalité entre les variations de densité optiques depuis l'origine et les variations d'intensité de fluorescence également depuis l'origine. Il a l'avantage d'être simple et de ne nécessiter aucune analyse préalable du comportement du réactif dans le temps.

Le procédé peut être généralisé à un réactif comprenant plusieurs composés fluorescents, avec des pics d'absorption à des longueurs d'onde différentes et éventuellement des caractéristiques de dégradation différentes.

Dans ce cas, on peut :

- effectuer des mesures de densités optiques initiales $DO_i(0)$ et des mesures de densités optiques courantes $DO_i(t)$ à plusieurs groupes de longueurs d'onde $\lambda_i$,
- calculer des rapports $K_i(t) = DO_i(0) / DO_i(t)$ à ces longueurs d'onde, et
- appliquer des corrections distinctes aux différentes mesures de fluorescence $FLUO_{ci}(t) = K_i(t) \times FLUO_{mi}(t)$ correspondant aux différents composés fluorescents.

### Second modèle de correction

Ce modèle de correction est basé sur la détermination préalable d'un modèle de dégradation du réactif. Cette détermination peut être effectuée à partir de mesures expérimentales d'absorbance DO et de niveaux de fluorescence FLUO sur des cellules de référence, effectuées au cours du vieillissement du réactif.

Le modèle de dégradation permet d'exprimer une variation de niveau de fluorescence $\Delta$FLUO en fonction d'une variation de la densité optique du réactif $\Delta$DO :

$$\Delta\text{FLUO} = f(\Delta\text{DO}).$$

En général, le modèle de dégradation peut être exprimé sous la forme d'un coefficient linéaire :

$$a = \Delta\text{FLUO} / \Delta\text{DO}.$$

Ensuite, de la même manière que pour le premier modèle de correction, on effectue une mesure de la densité optique initiale du réactif pris isolément à sa date de fabrication, donc sans dégradation. Cette quantité est notée DO(0). Cette mesure peut être effectuée par exemple lorsque le réactif est réceptionné, ou lors de sa fabrication.

Lors de l'utilisation de l'analyseur, on effectue des mesures de la densité optique courante DO(t) du réactif pris isolément à un instant t donné. Ces mesures sont effectuées de préférence à un instant t proche du moment où sont effectuées les mesures de fluorescence, notées FLUOm(t), c'est-à-dire dans la même journée ou la même semaine. Elles peuvent être effectuées par exemple lors d'un cycle de maintenance ou de mise en route journalier ou hebdomadaire de l'analyseur sanguin.

On calcule un nombre de canaux (ou de niveaux) de fluorescence perdus :

$$\text{CP}(t) = f(\text{DO}(0) - \text{DO}(t)),$$

ou, dans le cas d'un coefficient linéaire :

$$\text{CP}(t) = (\text{DO}(0) - \text{DO}(t)) \times a.$$

On peut ensuite calculer ensuite un gain correctif :

$$\text{GC}(t) = <\text{FLUO}(t)> / (<\text{FLUO}(t)> - \text{CP}(t)).$$

La grandeur <FLUO(t)> est une valeur de référence de fluorescence qui peut être, de manière non limitative, une valeur médiane ou moyenne des mesures, ou une valeur correspondant à une population de cellules particulière.

Le gain correctif GC(t) est ensuite appliqué au signal de fluorescence mesuré à la date t d'utilisation du réactif, FLUOm(t), ce qui permet de calculer le niveau de fluorescence corrigé,

$$FLUOc(t) = GC(t) \times FLUOm(t).$$

Le procédé peut être généralisé à la mesure de plusieurs densités optiques notées DOi (i compris entre 1 et n) pour des groupes de longueurs d'onde allant de λi à λn.

On peut alors déterminer n fonctions notées fi(ΔDOi) permettant de calculer des corrections distinctes pour différentes longueurs d'onde de fluorescence. Ces corrections s'écrivent sous la forme :

$$CPi(t) = fi(DOi(0) - DOi(t)), \text{ i étant compris entre 1 et n.}$$

i étant compris entre 1 et n.

A titre d'exemples non limitatifs, un réactif composé de deux composés fluorescents peut être basé sur le Thiazole Orange et le composé Hoescht® 33342 qui est un colorant à haute affinité ADN. Un ou plusieurs composés fluorescents peuvent également être choisis parmi la liste non exhaustive suivante : SYTOX Green, série Vybrant, YOYO-1, la série SYTO, la série Hoechst, l'Acridine Orange ou SYBR Green 1.

Nous allons maintenant décrire plus précisément un exemple d'application du second modèle de correction au Thiazole Orange.

Les mesures de densité optique sont effectuées dans une chambre de mesure spectrophotométrique. La cuve de mesure est traversée par un faisceau de lumière issu d'une diode électroluminescente de type NICHIA® NSP590S (Vert-bleuté), dont la longueur d'onde centrale d'émission est de 501 nm pour une intensité de i=30 mA. En réception, on utilise une photodiode OSRAM® BPW21 adaptée aux applications entre 350 nm et 820nm dont la sensibilité spectrale est 0.34 A/W à 550 nm.

Afin de bâtir un modèle de correction précis, des mesures de densité optique ont été effectuées pour des réactifs âgés respectivement de 3 semaines (S3), 5 semaines (S5), 13 semaines (S13), 32 semaines (S32), 52 semaines (S52) et 77 semaines (S77).

On considère que la valeur de densité optique à t=0 notée DO(0) est celle correspondant à S3.

On en déduit la différence ΔDO(t) entre la densité optique initiale DO(0) et chacune des densités optiques DO(t) mesurées au cours du temps.

Voici les résultats :

| Semaines | jours | DO | ΔDO mesurée |
|---|---|---|---|
| 3 | 21 | 0.2943 | 0 |
| 5 | 35 | 0.2956 | -0.0013 |
| 13 | 91 | 0.2835 | 0.0108 |
| 32 | 224 | 0.2401 | 0.0542 |
| 52 | 364 | 0.2197 | 0.0746 |
| 77 | 539 | 0.1936 | 0.1007 |

On peut ainsi construire un modèle d'interpolation qui permet de calculer ΔDO à tout instant :

$\Delta DO = -2 \cdot 10^{-7} t^2 + 0.003 \, t - 0.0104$, avec t en jours.

Ce modèle présente un très bon coefficient de corrélation ($R^2 = 0.9918$).

Des mesures de fluorescence ont par ailleurs été effectuées sur trois échantillons de sangs frais. Pour chaque sang frais, la moyenne de l'intensité de fluorescence <FLUO(t)> des neutrophiles a été calculée.

Les neutrophiles ont été choisis parce qu'ils donnent un nuage de points bien identifiable et peu dispersé lors des mesures (nuage de points 20 de la figure 3). On sait que la moyenne de l'intensité de fluorescence pour les échantillons de sang frais doit être réglée à 1100 canaux (d'intensité) pour une température de 35°.

On peut ainsi évaluer pour ces échantillons de sangs frais, la perte de fluorescence (ΔFLUO mesuré) en fonction de l'âge du réactif. Les résultats sont les suivants :

| âge du réactif | âge du réactif en jour | ΔDO calculée | ΔFLUO mesuré |
|---|---|---|---|
| S7 | 49 | 0.0043 | 30 |
| M7 | 212 | 0.0477 | 150 |
| M11 | 334 | 0.0727 | 162 |
| M16 | 486 | 0.0956 | 286 |

La variation de densité optique ΔDO est calculée avec le modèle d'interpolation.

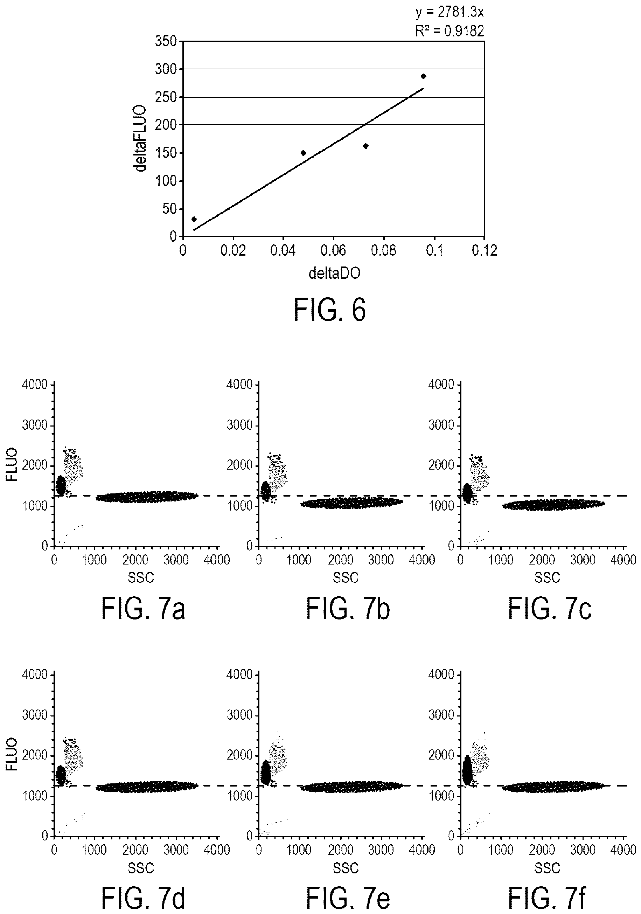

En référence à la figure 6, on établit donc la corrélation entre les variations de niveaux de fluorescence et les variations de densité optique et on peut en déduire le coefficient $\alpha = \Delta FLUO / \Delta DO$ du modèle de dégradation.

Ici on obtient $\alpha = 3000$.

La compensation des mesures est effectuée comme suit.

Pour chaque réactif, il convient de faire une mesure de densité optique DO(0) au plus près de sa date de production. Dans notre expérience, DO(0)=0.2385.

Ensuite, une mesure de densité optique DO(t) de chacun des réactifs est effectuée avant le passage d'une série d'échantillons.

On calcule le nombre de canaux perdus en fluorescence :

$$CP(t) = (DO(0) - DO(t)) \times 3000.$$

On considère que ce nombre de canaux perdus est équivalent à la perte sur la moyenne en intensité de fluorescence des neutrophiles <FLUO(t)>.

L'intensité de fluorescence moyenne des neutrophiles étant de 1100, on en déduit un gain correctif pour l'ensemble des cellules :

$$GC(t) = 1100 / (1100 - CP(t)).$$

La figure 7 illustre un exemple de compensation. Dans les figures 7a à 7f, chaque point représente une cellule. Les points sont positionnés selon des axes correspondant, respectivement, en abscisses à des mesures de diffusion orthogonale (SSC), et en ordonnées à des mesures de fluorescence orthogonale (FLUO), effectuées simultanément et selon une direction perpendiculaire aux directions d'illumination et de flot de cellules.

Les figures 7a, 7b et 7c présentent des mesures sans compensation, avec un réactif respectivement en sortie de production, âgé de 9 mois (M9) et âgé de 13 mois (M13). On observe bien un décalage des niveaux de fluorescence.

Les figures 7d, 7e et 7f présentent des mesures avec la compensation telle que décrite précédemment, avec un réactif respectivement en sortie de production, âgé de 9 mois (M9) et âgé de 13 mois (M13). Les niveaux de fluorescence corrigée restent effectivement constants.

Les méthodes de compensation selon l'invention sont notamment très utiles pour les échantillons de sang sur lesquels l'utilisation de seuils fixes est indispensable (par exemple s'il y a trop peu de cellules, ou pas de population marquée). En effet, l'utilisation de seuils fixes impose d'être extrêmement précis sur la fluorescence étant donné que c'est à partir de la fluorescence que l'on détermine le degré de maturité des cellules et donc la détection d'une éventuelle pathologie. Ainsi, une incertitude sur la fluorescence des cellules pourrait par exemple conduire à une non-détection de cellules blastiques. Cette méthode de compensation est donc indispensable pour éviter des faux négatifs.

Le procédé selon l'invention est aisément intégrable dans un dispositif d'analyse biologique, ou d'analyse sanguine.

Sa mise en oeuvre peut être largement automatisée en ajoutant au dispositif une cuve de mesure spectrophotométrique dans laquelle les mesures de densité optique du ou des réactifs sont effectuées.

Pour effectuer des mesures à différentes longueurs d'onde, on peut avantageusement utiliser une source de lumière polychromatique et un photorécepteur doté d'un système de filtrage optique permettant la mesure des densités optiques aux diverses longueurs d'onde centrales $\lambda i$ (i compris entre 1 et n).

Nous allons maintenant présenter un exemple de mise en oeuvre du procédé selon l'invention dans un dispositif d'analyse biologique ou d'analyse sanguine.

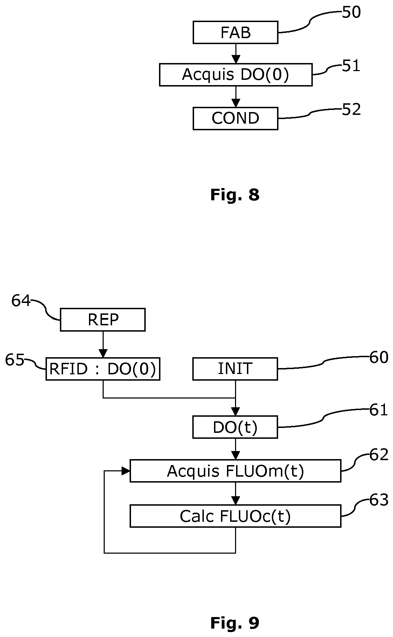

En référence à la figure 8, dans un premier temps, le réactif est produit (étape 50) puis conditionnés dans des contenant tels que des bouteilles (étape 52).

Comme expliqué précédemment, le procédé selon l'invention comprend en outre une étape 51 de mesure de la densité optique initiale DO(0) du réactif, qui est effectuée avant ou lors du conditionnement 52.

La valeur de la densité optique initiale DO(0) mesurée est mémorisée par exemple dans un tag RFID fixé sur le contenant, pour son utilisation ultérieure.

En référence à la figure 9, dans un deuxième temps, les bouteilles de réactif sont insérées (étape 64) dans un dispositif d'analyse biologique ou d'analyse sanguine, en vue de l'utilisation du réactif. Le tag RFID est alors lu (étape 65) et la valeur de densité optique initiale DO(0) du réactif contenu dans cette bouteille est mémorisée dans le dispositif.

De manière habituelle, l'exécution de mesures avec un dispositif d'analyse biologique ou d'analyse sanguine nécessite une étape préalable d'initialisation 60, qui est effectuée par exemple quotidiennement. Cette étape d'initialisation 60 comprend des opérations habituelles de contrôle, réglages, rinçage, ... pour mettre le dispositif en état pour effectuer des mesures.

Conformément à l'invention, on effectue alors également une étape 61 de mesure de la densité optique courante DO(t) du réactif. On obtient ainsi une valeur de densité optique courante DO(t) qui est utilisée pour corriger les mesures jusqu'à l'exécution d'une nouvelle étape d'initialisation 60, ou jusqu'au remplacement 64 de la bouteille de réactif si ce dernier intervient avant.

En cas de remplacement 64 d'une bouteille de réactif, une mesure 61 de la densité optique courante DO(t) du nouveau réactif est effectuée avant son utilisation.

On peut ensuite effectuer des cycles de mesure sur des échantillons biologiques ou sanguins.

Ces cycles de mesure comprennent :

- une acquisition 62 de mesures de signal de fluorescence FLUOm(t) ;
- une correction 63 des effets de la dégradation du réactif, pour obtenir des valeurs corrigées FLUOc(t). les valeurs corrigées FLUOc(t) sont calculées selon les modèles de correction décrits précédemment, en utilisant les valeurs de densité optique initiale DO(0) et de densité optique courante DO(t) mémorisées.

Du fait de la répétition périodique des étapes d'initialisation 60, les mesures 61 de densité optique courante DO(t) peuvent être considérées comme étant réalisées à des instants suffisamment proches des instants où sont effectuées les mesures de fluorescences FLUOm(t).

Bien sûr, l'invention n'est pas limitée aux exemples qui viennent d'être décrits et de nombreux aménagements peuvent être apportés à ces exemples sans sortir du cadre de l'invention. défini par les revendications suivantes.

## Revendications

1. Procédé pour compenser les variations de mesure de fluorescence dues à la dégradation d'un réactif stocké en phase aqueuse, lequel réactif comportant au moins un composé fluorescent et permettant l'identification de particules, lequel procédé comprenant une étape de mesure (62) de niveau de fluorescence FLUOm(t) sur des particules marquées par ledit réactif, et étant **caractérisé en ce qu'**il comprend en outre des étapes :

   - d'exécution de mesures d'absorbance (61) à au moins une longueur d'onde sur une solution dudit réactif, à un instant t proche de celui desdites mesures de niveau de fluorescence FLUOm(t), de telle sorte à déterminer au moins une densité optique courante DO(t) du réactif, et,
   - de calcul d'une correction (63) desdites mesures de niveau de fluorescence FLUOm(t) de sorte à déterminer des niveaux de fluorescence corrigés FLUOc(t) en utilisant ladite ou lesdites densité(s) optique(s) courante(s) DO(t) et au moins une densité optique initiale DO(0) du réactif exempt de dégradation.

2. Le procédé de la revendication 1, dans lequel le réactif comprend un au moins un composé fluorescent de type polyméthine.

3. Le procédé de l'une des revendications 1 ou 2, dans lequel le réactif comprend du Thiazole Orange.

4. Le procédé de l'une des revendications 1 à 3, qui comprend en outre des étapes de :

   - calcul d'un rapport K(t) correspondant au ratio entre une densité optique initiale DO(0) et une densité optique courante DO(t), et
   - calcul de niveaux de fluorescence corrigés FLUOc(t) en multipliant des niveaux de fluorescence FLUOm(t) mesurés vers l'instant de mesure t sur des particules marquées par le réactif, par le rapport K(t).

5. Le procédé de la revendication 4, qui comprend en outre des étapes de :

- mesure de densités optiques initiales DOi(0) et de densités optiques courantes DOi(t) à plusieurs longueurs d'onde λi,
- calcul de rapports Ki(t) auxdites longueurs d'onde λi, et
- calcul de niveaux de fluorescence corrigés FLUOci(t) auxdites longueurs d'onde λi.

6. Le procédé de l'une des revendications 1 à 3, qui comprend en outre des étapes de :

- détermination au préalable d'un modèle de dégradation établissant une relation entre des variations de niveau de fluorescence ΔFLUO sur des particules de référence et des variations de la densité optique du réactif ΔDO du fait de sa dégradation, et
- calcul d'un nombre de canaux de fluorescence perdus CP(t), à partir de mesures de densité optique courante DO(t), de mesures de densité optique initiale DO(0), et dudit modèle de dégradation.

7. le procédé de la revendication 6, qui comprend en outre des étapes de :

- détermination au préalable d'un modèle de dégradation sous la forme d'un coefficient α correspondant à un ratio entre des variations de niveau de fluorescence ΔFLUO et des variations de densité optique du réactif ΔDO,
- calcul d'un nombre de canaux de fluorescence perdus CP(t), sous la forme d'une différence entre la densité optique courante DO(t) et la densité optique initiale DO(0), multipliée par le coefficient α,
- détermination d'une valeur de référence de fluorescence <FLUO(t)> statistiquement représentative des niveaux de fluorescence FLUOm(t) mesurés,
- calcul d'un gain correctif GC(t) correspondant à une valeur de référence de fluorescence <FLUO(t)> divisée par une différence entre ladite valeur de référence de fluorescence <FLUO(t)> et le nombre de canaux de fluorescence perdus CP(t), et
- calcul de niveaux de fluorescence corrigés FLUOc(t) en multipliant des niveaux de fluorescence FLUOm(t) mesurés vers un instant de mesure t sur des particules marquées par le réactif, par le gain correctif GC(t).

8. le procédé de l'une des revendications 6 ou 7, qui comprend en outre une détermination d'une pluralité de modèles de dégradation à partir de mesures de densités optiques à plusieurs longueurs d'onde.

9. Dispositif d'analyse cellulaire par cytométrie en flux, comprenant des moyens de mesure de niveau de fluorescence sur des particules marquées par un réactif, **caractérisé en ce qu'**il comprend en outre :

- des moyens pour effectuer des mesures d'absorbance à au moins une longueur d'onde sur une solution dudit réactif, à un instant t proche de celui desdites mesures de niveau de fluorescence, de telle sorte à déterminer au moins une densité optique courante DO(t) du réactif, et
- des moyens de calcul aptes à calculer une correction desdites mesures de niveau de fluorescence FLUOm(t) de sorte à déterminer des niveaux de fluorescence corrigés FLUOc(t) en utilisant ladite ou lesdites densité(s) optique(s) courante(s) DO(t) et au moins une densité optique initiale DO(0) du réactif exempt de dégradation.

10. Le dispositif d'analyse cellulaire de la revendication 9, qui comprend une cuve de mesure spectrophotométrique.

11. Le dispositif d'analyse cellulaire de la revendication 10, dans lequel la cuve de mesure spectrophotométrique comprend une source de lumière polychromatique et un photorécepteur doté d'un système de filtrage optique permettant la mesure des densités optiques aux diverses longueurs d'onde centrales λi.

12. Le dispositif selon l'une des revendications 9 à 11, qui comprend en outre un analyseur d'hématologie.

**Patentansprüche**

1. Verfahren zur Kompensation der Variationen der Fluoreszenzmessung, die auf den Abbau eines Reagens zurückzuführen sind, das in wässriger Phase aufbewahrt wird, wobei das Regens mindestens eine fluoreszierende Verbindung umfasst und die Identifikation von Partikeln gestattet, wobei das Verfahren einen Schritt (62) zur Messung des Fluoreszenzgrads FLUOm(t) an den Partikeln umfasst, die mit dem Reagens markiert werden, und **dadurch gekennzeichnet ist, dass** dieses außerdem die Schritte umfasst:

- Ausführung von Messungen des Absorptionsvermögens (61) bei mindestens einer Wellenlänge an einer Lösung des Reagens zu einem Zeitpunkt t nahe bei jenem der Messungen des Fluoreszenzgrads FLUOm(t), um mindestens eine aktuelle optische Dichte DO(t) des Reagens zu bestimmen, und

- Berechnung einer Korrektur (63) der Messungen des Fluoreszenzgrads FLUOm(t), um korrigierte Fluoreszenzgrade FLUOc(t) zu bestimmen, unter Verwendung der aktuellen optischen Dichte(n) DO(t) und mindestens einer anfänglichen optischen Dichte DO(0) des Reagens ohne Abbau.

2. Verfahren nach Anspruch 1,
wobei das Reagens mindestens eine fluoreszierende Verbindung des Typs Polymethin umfasst.

3. Verfahren nach einem der Ansprüche 1 oder 2,
wobei das Reagens Thiazol-Orange umfasst.

4. Verfahren nach einem der Ansprüche 1 bis 3,
welches außerdem die Schritte umfasst:

- Berechnung eines Verhältnisses K(t), das dem Verhältnis zwischen einer anfänglichen optischen Dichte DO(0) und einer aktuellen optischen Dichte DO(t) entspricht, und
- Berechnung der korrigierten Fluoreszenzgrade FLUO(t) durch Multiplikation der Fluoreszenzgrade FLUOm(t), die zum Messzeitpunkt t an den mit dem Reagens markierten Partikeln gemessen wurden, mit dem Verhältnis K(t).

5. Verfahren nach Anspruch 4,
welches außerdem die Schritte umfasst:

- Messung der anfänglichen optischen Dichten DOi(0) und der aktuellen optischen Dichten DOi(t) bei mehreren Wellenlängen $\lambda i$,
- Berechnung der Verhältnisse Ki(t) bei den Wellenlängen $\lambda i$, und
- Berechnung der korrigierten Fluoreszenzgrade FLUOci(t) bei den Wellenlängen $\lambda i$.

6. Verfahren nach einem der Ansprüche 1 bis 3,
welches außerdem die Schritte umfasst:

- vorherige Bestimmung eines Abbaumodells, das eine Beziehung zwischen den Variationen des Fluoreszenzgrads $\Delta$FLUO an den Referenzpartikeln und den Variationen der optischen Dichte des Reagens $\Delta$DO aufgrund seinem Abbau herstellt, und
- Berechnung einer Anzahl verlorener Fluoreszenzkanäle CP(t) aus den Messungen der aktuellen optischen Dichte DO(t), den Messungen der anfänglichen optischen Dichte DO(0) und dem Abbaumodell.

7. Verfahren nach Anspruch 6,
welches außerdem die Schritte umfasst:

- vorherige Bestimmung eines Abbaumodells in der Form eines Koeffizienten $\alpha$, der einem Verhältnis zwischen den Variationen des Fluoreszenzgrads $\Delta$FLUO und den Variationen der optischen Dichte des Reagens $\Delta$DO entspricht,
- Berechnung einer Anzahl verlorener Fluoreszenzkanäle CP(t) in der Form einer Differenz zwischen der aktuellen optischen Dichte DO(t) und der anfänglichen optischen Dichte DO(0), multipliziert mit dem Koeffizienten $\alpha$,
- Bestimmung eines Fluoreszenzreferenzwerts <FLUO(t)>, der statistisch für die gemessenen Fluoreszenzgrade FLUOm(t) repräsentativ ist,
- Berechnung einer Korrekturverstärkung GC(t), die einem Fluoreszenzreferenzwert <FLUO(t)> entspricht, dividiert durch eine Differenz zwischen dem Fluoreszenzreferenzwert <FLUO(t)> und der Anzahl verlorener Fluoreszenzkanäle CP(t), und
- Berechnung der korrigierten Fluoreszenzgrade FLUOc(t) durch Multipliation der Fluoreszenzgrade FLUOm(t), die zu einem Messzeitpunkt t an den mit dem Reagens markierten Partikeln gemessen wurden, mit der Korrekturverstärkung GC(t).

8. Verfahren nach einem der Ansprüche 6 oder 7,
welches außerdem eine Bestimmung einer Vielzahl von Abbaumodellen aus Messungen der optischen Dichten bei mehreren Wellenlängen umfasst.

9.  Vorrichtung zur zellulären Analyse gemäß Durchflusszytometrie, umfassend Mittel zur Messung des Fluoreszenzgrads an Partikeln, die mit einem Reagens markiert sind, **dadurch gekennzeichnet, dass** diese außerdem umfasst:

    - Mittel zur Durchführung von Messungen des Absorptionsvermögens bei mindestens einer Wellenlänge an einer Lösung des Reagens zu einem Zeitpunkt t nahe bei jenem der Messungen des Fluoreszenzgrads, um mindestens eine aktuelle optische Dichte DO(t) des Reagens zu bestimmen, und
    - Mittel zur Berechnung, die geeignet sind, eine Korrektur der Messungen des Fluoreszenzgrads FLUOm(t) zu berechnen, um korrigierte Fluoreszenzgrade FLUOc(t) zu bestimmen, unter Verwendung der aktuellen optischen Dichte(n) DO(t) und mindestens einer anfänglichen optischen Dichte DO(0) des Reagens ohne Abbau.

10. Vorrichtung zur zellulären Analyse nach Anspruch 9,
    welche einen Behälter zur spektrophotometrischen Messung umfasst.

11. Vorrichtung zur zellulären Analyse nach Anspruch 10,
    wobei der Behälter zur spektrophotometrischen Messung eine polychromatische Lichtquelle und einen Photoempfänger umfasst, dotiert mit einem optischen Filtersystem, das die Messung optischer Dichten bei verschiedenen zentralen Wellenlängen $\lambda i$ gestattet.

12. Vorrichtung nach einem der Ansprüche 9 bis 11,
    welche außerdem einen Hämatologieanalysator umfasst.

**Claims**

1.  Method for compensating for fluorescence measurement variations due to the degradation of a reagent stored in aqueous phase, which reagent comprising at least one fluorescent compound and allowing particles to be identified, which method comprising a step (62) of measuring fluorescence level FLUOm(t) on particles labelled with said reagent, and being **characterized in that** it furthermore comprises:

    - a step (61) of carrying out absorbance measurements at at least one wavelength on a solution of said reagent, at a time t close to that of said measurements of fluorescence level FLUOm(t), in such a way as to determine at least one current optical density DO(t) of the reagent, and,
    - a step (63) of calculating a correction of said measurements of fluorescence level FLUOm(t) so as to determine corrected fluorescence levels FLUOc(t) using said one or more current optical densities DO(t) and at least one initial optical density DO(0) of the reagent exempt from degradation.

2.  Method according to Claim 1, wherein the reagent comprises at least one fluorescent compound of polymethine type.

3.  Method according to either of Claims 1 and 2, wherein the reagent comprises thiazole orange.

4.  Method according to one of Claims 1 to 3, which method furthermore comprises steps of:

    - calculating a ratio K(t) corresponding to the ratio between an initial optical density DO(0) and a current optical density DO(t), and
    - calculating corrected fluorescence levels FLUOc(t) by multiplying the fluorescence levels FLUOm(t) measured toward the measurement time t on particles labelled with the reagent, by the ratio K(t).

5.  The method according to Claim 4, which method furthermore comprises steps of:

    - measuring initial optical densities DOi(0) and current optical densities DOi(t) at a plurality of wavelengths $\lambda i$,
    - calculating ratios Ki(t) at said wavelengths $\lambda i$, and
    - calculating corrected fluorescence levels FLUOci(t) at said wavelengths $\lambda i$.

6.  Method according to one of Claims 1 to 3, which method furthermore comprises steps of:

    - determining beforehand a degradation model establishing a relationship between variations $\Delta$FLUO in fluo-

rescence level on reference particles and variations ∆DO in the optical density of the reagent because of its degradation, and
- calculating a number CP(t) of lost fluorescence channels, on the basis of measurements of current optical density DO(t), of measurements of initial optical density DO(0), and of said degradation model.

7. Method according to Claim 6, which method furthermore comprises steps of:

- determining beforehand a degradation model taking the form of a coefficient $\alpha$ corresponding to a ratio between variations ∆FLUO in fluorescence level and variations ∆DO in the optical density of the reagent,
- calculating a number CP(t) of lost fluorescence channels, taking the form of a difference between the current optical density DO(t) and the initial optical density DO(0), multiplied by the coefficient $\alpha$,
- determining a reference fluorescence value <FLUO(t)> that is statistically representative of the measured fluorescence levels FLUOm(t),
- calculating a corrective gain GC(t) corresponding to a reference fluorescence value <FLUO(t)> divided by a difference between said reference fluorescence value <FLUO(t)> and the number CP(t) of lost fluorescence channels, and
- calculating corrected fluorescence levels FLUOc(t) by multiplying the fluorescence levels FLUOm(t) measured toward a measurement time t on particles labelled by the reagent, by the corrective gain GC(t).

8. Method according to either of Claims 6 or 7, which method furthermore comprises determining a plurality of degradation models on the basis of measurements of optical densities at a plurality of wavelengths.

9. Device for cellular analysis by flow cytometry, comprising means for measuring fluorescence levels on particles labelled with a reagent, **characterized in that** it furthermore comprises:

- means for carrying out absorbance measurements at at least one wavelength on a solution of said reagent, at a time t close to that of said measurements of fluorescence level, in such a way as to determine at least one current optical density DO(t) of the reagent, and
- calculating means able to calculate a correction of said measurements of fluorescence level FLUOm(t) so as to determine corrected fluorescence levels FLUOc(t) using said one or more current optical densities DO(t) and at least one initial optical density DO(0) of the reagent exempt from degradation.

10. Device for cellular analysis according to Claim 9, which device comprises a spectrophotometric measurement cell.

11. Device for cellular analysis according to Claim 10, wherein the spectrophotometric measurement cell comprises a polychromatic light source and a photoreceiver equipped with an optical filtering system allowing the optical densities to be measured at various central wavelengths $\lambda i$.

12. Device according to one of Claims 9 to 11, which device furthermore comprises a haematology analyser.

FIG. 1

FIG. 2a

FIG. 2b

FIG. 2c

(1248,2468)

(992,2720)

FIG. 3a

FIG. 3b

FIG. 4

FIG. 5

FIG. 6

FIG. 7a

FIG. 7b

FIG. 7c

FIG. 7d

FIG. 7e

FIG. 7f

FAB — 50

↓

Acquis DO(0) — 51

↓

COND — 52

**Fig. 8**

64 — REP

↓

65 — RFID : DO(0)          INIT — 60

↓

DO(t) — 61

↓

Acquis FLUOm(t) — 62

↓

Calc FLUOc(t) — 63

**Fig. 9**

18

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2821428
- WO 2006024716 A
- US 7638290 B
- EP 2175340 A
- FR 2873813

**Littérature non-brevet citée dans la description**

- **R. HULSPAS et al.** Flow cytometry and the stability of phycoerythrin-tandem dye conjugates. *CYTOMETRY PART A,* 23 Septembre 2009, vol. 75A (11